# EUROPEAN PATENT SPECIFICATION

(11) **EP 3 665 269 B1**
(45) Date of publication and mention of the grant of the patent: **15.09.2021**
(21) Application number: 18756094.1
(22) Date of filing: 01.08.2018
(51) Int. Cl.: C12N 5/00, G01N 27/00, C07K 16/28

(54) **USE OF MAGNETIC CELLS TO MANIPULATE NON-MAGNETIC CELLS**
VERWENDUNG VON MAGNETISCHEN ZELLEN ZUR MANIPULATION NICHTMAGNETISCHER ZELLEN
UTILISATION DE CELLULES MAGNÉTIQUES POUR LA MANIPULATION DE CELLULES NON MAGNÉTIQUES

(30) Priority: 08.08.2017 US 201762542745 P
(43) Date of publication of application: 17.06.2020
(73) Proprietor: Greiner Bio-One North America, Inc., Monroe NC 28110 (US)
(72) Inventor: SOUZA, Glauco, R., Monroe, NC 28110 (US)
(74) Representative: Schrell, Andreas
(86) International application number: PCT/US2018/044806
(87) International publication number: WO 2019/032345

(56) References cited:
- EP-A1- 1 650 293
- WO-A1-2010/036957
- WO-A1-2011/038370
- WO-A1-2013/188449
- MARGOLIS L B ET AL: "Magnetoliposomes: Another principle of cell sorting", BIOCHIMICA ET BIOPHYSICA ACTA (BBA) - BIOMEMBRANES, ELSEVIER, AMSTERDAM, NL, vol. 735, no. 1, 26 October 1983 (1983-10-26), pages 193-195, XP023360500, ISSN: 0005-2736, DOI: 10.1016/0005-2736(83)90276-6 [retrieved on 1983-10-26]
- GLAUCO R. SOUZA ET AL: "Three-dimensional tissue culture based on magnetic cell levitation", NATURE NANOTECHNOLOGY, vol. 5, no. 4, 14 March 2010 (2010-03-14), pages 291-296, XP55233171, GB ISSN: 1748-3387, DOI: 10.1038/nnano.2010.23

## Description

### PRIOR RELATED APPLICATIONS

### FIELD OF THE INVENTION

The invention relates to materials, methods of making, and methods of using magnetic cells as a tool to collect and manipulate other cells, as wells as to a magnetic cell complex comprising at least one magnetized display cell and at least one target cell.

### BACKGROUND THE INVENTION

The use of magnetic beads to select and enrich cells has long been known. Indeed, there are several commerical suppliers of magentic bead-based cell sorting technologies, including Mojosort, EasySep, MACS MicroBead Technology, Dynabeads, to name just a few. Thus, the technology is well developed and well known in the art.

There are many variations on magnetic cell separation techniques, but one important method uses antibodies to target the cells of interest, and then a magnetic field to collect those targeted cells, usually via a second antibody that is conjugated to a magnetic bead.

Usually an antibody cocktail will bind either the cell of interest (positive selection) or the cells of non-interest (negative selection). After a short incubation the addition of magnetic nanoparticle beads to the cell mixture then binds the antibodies from the previous incubation, often via a secondary antibody that has been conjugated to the magnetic bead. After another short incubation, cells can then be placed into a magnetic field. After a few minutes, the antibody bound cells will be drawn towards the magnet, either the bound or the the unbound cells can be collected. This technology allows rapid and easy isolation of cell populations from bulk populations, and a variety of applications have been developed based on these basic principles.

However, magnetic antibody based cell isolation involves some upfront investment in the purchasing of magnets (approaching $1000) and antibody kits (ranging from $300-$700). In addition, the beads are not natural and thus are either removed for further culturing or are retained, but contribute to the artificiality of the environment. Thus, although these bead-based approaches are very useful, there is still room for improved methods of isolating cells that avoids the use of expensive magnetically conjugated antibody beads, avoids the use of artificial bead materials and chemicals, and provides the best cell collection possible, for further techniques such as assay or culturing, especially 3D culturing.

### SUMMARY OF THE INVENTION

Herein, we present a new use for magnetized cells or small cluster of magnetized cells assembled in 3D, wherein the cells are engineered to display anti-target antibodies that will bind to target cells. These magnetized display cells can be used in place of magnetic beads for cell enrichment. Thus, the beads are no longer needed, nor is a second antibody needed.

The magnetized display cells preferably display antibodies or antigen binding portions thereof on their surfaces, but any other target specific receptor, ligand, chemical or one member of a binding pair could be used. Such "display surface markers" can be native, genetically engineered, overexpressed, or can be chemically added to cells. In addition, the magnetized cells of specific organs that are metastasis targets of specific cancer cell types could be used, as could irradiated cells or non-proliferating cells. The point being that the magnetized display cells carry particular chemical or physical characteristics in order to target the cells of interest, thereby binding to same. All such cells are called "magnetized display cells" herein.

The magnetized display cells can also carry fluoresent molecules or atoms, e.g. green fluorescence proteins (GFP), or other labels, that can be used to identify cell-cell binding events or can be used to facilitate cell sorting. These cells are called "magnetized labeled display cells" and could also be called called "magnetized fluorescent display cells" where the label is a fluorescent marker.

To the extent that fibroblast cells are used as magnetized display cells, they can then be retained in the culture for use as e.g., feeder cells or native stroma surrogate. This is particularly beneficial in the case of 3D cultures, which benefit from culturing with feeder cells e.g. when conditionally reprograming cells and culturing circulating tumor cells, stem cells, and primary cells.

Furthermore, to the extent that immune cells are used as magnetized display cells, they can then be retained in the culture for use as e.g. macrophage, T-cells, and antigen presenting cells. This is particularly beneficial in the case of capturing either antigen presenting cells and T-cells by magnetizing antigen presenting cells. This could also benefit the process of activating immune cells as well as capturing.

In order to prevent the fibroblast or immune cells from overtaking the cell culture, the selective agent is removed, and the cells, which are often genetically engineered to display surface markers, will slow their growth due to the expression burden, allowing the selected target cells to compete more effectively. Alternatively, other negative selective pressures could be used, such as the removal of an essential nutrient in a conditional mutant, temperature selection (temperature sensitive p-53 mutant and mutants of SV40), irradiation, and the like.

The creation of cells that display target antibodies on their surfaces is well known. Phage display has long been used for *in vitro* antibody affinity maturation and bacterial and yeast cell surface display systems have also been developed. "Mammalian cell display" systems have now been developed, thus potentially avoiding any steps need to transfer the selected antibody genes to another cell type. Any other method could be used as well.

In more detail, the mammalian cell display strategy developed by Ho, 2009, used human embryonic kidney 293T (HEK-293T) cells, but other cells types, especially fibroblast cells can be used. Mammalian cell display relies on the transient transfection of antibody encoding DNA to promote very high levels of antibody expression in mammalian cells. Moreover, the expressed mouse or human antibodies can contain the posttranslational modifications that are required for antibody function. It has been suggested that mammalian cell display could be used to express the recombinant antibody fragments that cannot be expressed in *E. coli.*

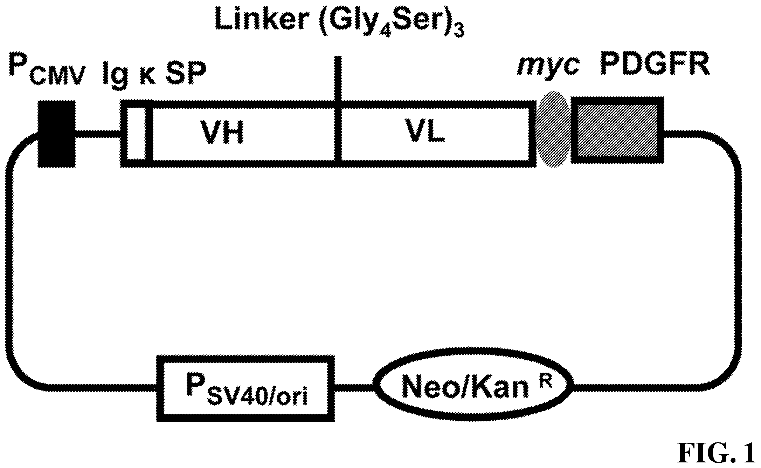

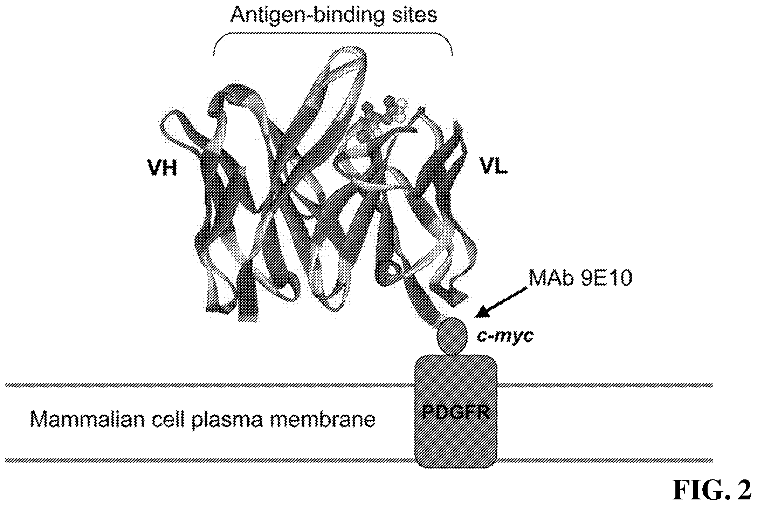

To display the Fv on the cell surface, the scFv is fused to the transmembrane domain of human platelet-derived growth factor receptor (PDGFR) (FIG. 1), although other transmembrane proteins could be used. The expression vector contained e.g., the cytomegalovirus promoter, the nucleotide sequence encoding the murine Igκ chain signal peptide (METDTLLLWVLLLWVPGSTGD), the scFv, a myc tag and the transmembrane domain (amino acids Ala513-Arg561) of PDGFR. The myc epitope tag at the carboxyl terminal of the scFv was used to measure the expression level. Ho expressed anti-CD22 (RFB4) scFv on HEK-293T cells **(****FIG. 2****).** Surface localization of the scFv-PDGFR fusion was verified by confocal fluorescence microscopy and flow cytometry. Cells labeled simultaneously with biotinylated CD22-Fc proteins and an anti-c-myc mAb were examined by laser scanning confocal microscopy (not shown).

Cells are then selected by e.g., FACS sorting, and the scFV genes recovered and placed into any desired cell type. Alternatively, the selected display cells can be magnetized as is, and directly used in the methods described herein.

Display cells are magnetized by incubation with a magnetic nanoparticle material, such as those described in WO2010036957 and WO2011038370 by Nano3D Biosciences®. Alternatively, a variation of the nanoparticle assembly is commercially available at Nano3D Biosciences® (Houston TX), under the trade name NANOSHUTTLE™. This new magnetic material provides a superior method of magnetizing cells without the use of any toxic or infectious agents, and the cells remain magnetized when the material is washed away.

The display cells could also be magnetized with any magnetic nanoparticle that can be taken up by the cell or otherwise introduced into the cell, e.g., by blasting, injection, electroporation, transduction, cationic liposomes, hydrogels, magnetic pressure, and the like. It is anticipated that any means of introduction will suffice, although NanoShuttle, may be the least cell perturbing method, and thus be preferred. Using NanoShuttle, the magnetic nanoparticles are taken up by the display cells, and retained for about 2 weeks. When using small cluster of magnetized display cells assembled in 3D, the magnetization can be retained for months. The cells thus are now magnetized, plus they display the antigen binding sites or other surface marker that will target the cell of interest.

The magnetic display cells are then combined with the target cells and incubated to allow antigen binding sites to bind to some epitope on the surface of the target cells. These cells can then be collected by the application of a strong magnetic field. The magnetic diplay cells can thus be used in any method where magnetic beads were previously used, including in cell manipulations, media changing, cell isolation or enrichment, cell expansion, 3D cell culture, cell levitation, cell aggregation, "ink" for bioprinting applications, and to localize magnetized display cells in vivo with the use of directed magnetic fields.

The general steps of the method include:
1. Selection of antibody or variable region displaying cells for the target cell of interest. This step may be optional if the antibody display cells or ligand display cells are already available, as is often the case. It may, however, be needed to transfer the requisite genes to another cell type, depending on the application.
2. Treating of display cells with NANOSHUTTLE™ or any another method, such that the cells uptake magnetic nanoparticles.
3. Incubate the magnetic display cell with target cells and allow the magnetic display cells to bind the target cells.
4. Collect magnetic cells and cells bound thereto with a magnetic field. The cells can be washed one or more times, media changed, and the like.
5. Optionally 2D or 3D cell culture of the magnetized display cells and target cell mixture.
6. Optionally perform whatever assay or imaging is of interest to the application, or use the 3D cell culture for *in vivo* therapeutics.
7. Optionally bioprinting of the magnetized display cells and target cell mixture.

The invention includes any one or more of the following embodiments, in all possible combinations:
- A method of isolating target cells from a mixture of cells, comprising:
   a) combining a mixture of cells comprising target cells and non-target cells with magnetized display cells that display one or more ligands on their surfaces, said ligands specific for a binding partner on a surface of said target cells;
   b) allowing said ligand to bind to said binding partner to produce magnetized display cells bound to said target cells;
   c) applying a magnetic field to collect said magnetized display cells bound to said target cells; and
   d) washing away non-target cells, thereby isolating said target cells.
- Any method herein, wherein said ligands comprising antigen binding sites and said binding partner comprises an antigen that is recognized by said antigen binding sites.
- Any method herein, wherein said magnetized display cells are fibroblasts or immune cells.
- Any method herein, wherein said magnetized display cells are from a fibroblast derived cell line.
- Any method herein, wherein said target cells and said magnetized display cells are human.
- Any method herein, comprising the further step of 3D culturing said magnetized display cells bound to said target cells.
- Any method herein, comprising the further step of 3D culturing said magnetized display cells bound to said target cells in a magnetic field such that said magnetized display cells bound to said target cells are levitated.
- Any method herein, comprising the further step of assaying said target cells.
- Any method herein, comprising the further step of bioprinting said magnetized display cells bound to said target cells.
- Any method herein, wherein said magnetized display cells are provided by treating cells displaying one or more ligands with magnetic nanoparticles.
- Any method herein, wherein said magnetic nanoparticles are introduced into said cells displaying one or more ligands by blasting, injection, electroporation, magnetic pressure, hydrogels, or cationic liposomes.
- Any method herein, wherein said magnetic nanoparticles are introduced into said cells displaying one or more ligands with a composition comprising:
   a) a negatively charged nanoparticle;
   b) a positively charged nanoparticle; and
   c) a support molecule,
   d) wherein one of said negatively charged nanoparticle or positively charged nanoparticle is a magnetically responsive element or compound, and wherein said support molecule holds said negatively charged nanoparticle and said positively charged nanoparticle in an intimate admixture forming a fibrous mat-like structure.
- A method of isolating target cells from a mixture of cells, comprising:
   a) combining cells displaying one or more antigen binding site(s) on their surfaces with a composition to form magnetized display cells, said composition comprising:
      i) a negatively charged nanoparticle;
      ii) a positively charged nanoparticle; and
      iii) a support molecule;
      iv) wherein one of said negatively charged nanoparticle or positively charged nanoparticle is a magnetically responsive element or compound, and wherein said support molecule holds said negatively charged nanoparticle and said positively charged nanoparticle in an intimate admixture forming a fibrous mat-like structure;
   b) combining a mixture of cells comprising target cells and non-target cells with said magnetized display cells;
   c) allowing said antigen binding site(s) to bind to one or more target cell(s) displaying an antigen that is recognized by said antigen binding site(s);
   d) applying a magnetic field to collect magnetized display cells bound to said target cells; and
   e) washing away non-target cells, thereby isolating magnetized display cells bound to said target cells.
- Any method disclosed herein, comprising the further step of separating the magnetized display cells from the target cells.
- Any method disclosed herein, wherein the further step of separating the magnetized display cells from the target cells comprises use of an excess of unbound ligand for competitive binding, use of enzymes that cleave specific sites of one member of the binding pair, in particular of the antigen or the antigen binding site(s), or use of enzymes that unspecifically cleave surface proteins of the members of the binding pair, in particular trypsin, papain, dispase, collagenase, hyaluronidase, or a mixture thereof.
- A method of isolating target cells from a mixture of cells, comprising:
   a) combining cells displaying one or more antigen binding site(s) on their surfaces with a composition to form magnetized display cells, said composition comprising:
      i) a negatively charged nanoparticle;
      ii) a positively charged nanoparticle; and
      iii) a support molecule;
      iv) wherein one of said negatively charged nanoparticle or positively charged nanoparticle is a magnetically responsive element or compound, and wherein said support molecule holds said negatively charged nanoparticle and said positively charged nanoparticle in an intimate admixture forming a fibrous mat-like structure;
   b) combining a mixture of cells comprising target cells and non-target cells with said magnetized display cells;
   c) allowing said antigen binding site(s) to bind to one or more target cell(s) displaying an antigen that is recognized by said antigen binding site(s);
   d) applying a magnetic field to collect magnetized display cells bound to said target cells; and
   e) washing away non-target cells, thereby isolating magnetized display cells bound to said target cells; and
   f) 3D culturing said isolating magnetized display cells bound to said target cells in a magnetic field sufficient to levitate said isolating magnetized cells bound to said target cells.
- Any method herein, a) wherein the support molecule comprises peptides, polysaccharides, nucleic acids, polymers, poly-lysine, fibronectin, collagen, laminin, BSA, hyaluronan, glycosaminoglycan, anionic, non-sulfated glycosaminoglycan, gelatin, nucleic acid, extracellular matrix protein mixtures, antibody, or mixtures or derivatives thereof, b) wherein said negatively charged nanoparticle is a gold nanoparticle, and c) wherein said positively charged nanoparticle is an iron oxide nanoparticle.
- A method of manipulating target cells, comprising:
   a) combining target cells with magnetized display cells that display one or more ligands on their surfaces, said ligands for specifically binding a target molecule on a surface of said target cells;
   b) allowing said ligands to bind to said target molecule to produce magnetized display cells bound to said target cells;
   c) applying a magnetic field to collect said magnetized display cells bound to said target cells, thereby allowing manipulation of said target cells.

In preferred embodiments, the cells are levitated with a composition comprising: a) a negatively charged nanoparticle; b) a positively charged nanoparticle; and c) a support molecule, wherein one of said negatively charged nanoparticle or positively charged nanoparticle contains a magnetically responsive material, such as iron or iron oxide, and wherein said support molecule holds said negatively charged nanoparticle and said positively charged nanoparticle in an intimate admixture.

Most preferred, the composition is NANOSHUTTLE™, which is known to be very effective and which is commercially available from Nano3D BioSciences (Houston TX).

Further, as described in prior patents, variously shaped 3D cultures can be made by modifying the shape of the magnetic field. For example, a ring magnet can be used to make a ring or donut shaped 3D culture. Magnetic fields can also be used to maintain or change such shape during the decellularization and recellularization processes.
- A magnetic cell complex, comprising:
   a) at least one magnetized display cell displaying at least one ligand on a surface of the at least one magnetized display cell; and
   b) at least one target cell having at least one binding partner on a surface of the at least one target cell;
   wherein said at least one ligand on the surfaces of the at least one magnetized display cell binds said binding partner on the surface of the at least one target cell so as to form the magnetic cell complex.
- Any magnetic cell complex herein, wherein said at least one ligand comprises antigen binding site(s) and said at least one binding partner comprises an antigen that is recognized by said antigen binding site(s).
- Any magnetic cell complex herein, wherein the at least one magnetized display cell comprises magnetic nanoparticles.
- Any magnetic cell complex herein, wherein the at least one magnetized display cell comprises magnetic nanoparticles, said magnetic nanoparticles comprising:
   i) a negatively charged nanoparticle;
   ii) a positively charged nanoparticle; and
   iii) a support molecule;
   iv) wherein one of said negatively charged nanoparticle or positively charged nanoparticle is a magnetically responsive element or compound, and wherein said support molecule holds said negatively charged nanoparticle and said positively charged nanoparticle in an intimate admixture forming a fibrous mat-like structure.
- Any magnetic cell complex herein, obtainable, in particular obtained, by any of the methods of isolating target cells from a mixture of cells herein.
- Any magnetic cell complex herein, obtainable, in particular obtained, by the method of manipulating target cells.

The following abbreviations may be used herein:

| Abbreviation | Definition |
|---|---|
| BAP | Bone alkaline phosphatase |
| BrEpic | Bronchial Epithelial |
| CHAPS | 3-[(3-cholamidopropyl)dimethylammonio]-1-propanesulfonate |
| DAPI | 4',6-diamidino-2-phenylindole-a fluorescent nucleic acid stain. |
| DMEM | Dulbecco's Modified Eagle's Medium |
| ECM | Extracellular matrix |
| EDTA | Ethylenediaminetetraacetic acid |
| FBS | Fetal Bovine Serum |
| HEK | Human Embryonic Kidney |
| HPF | human primary fibroblast |
| mECM | Magnetic ECM |
| mECM-HPF | Magnetic ECM from HPF cells |
| NNDF | human neonatal dermal fibroblasts |
| -NP | negatively charged nanoparticle |
| +NP | positively charged nanoparticle |
| NS | NANOSHUTTLE™ |
| P/S | Penicillin/Streptomycin |
| PBS | Phosphate buffered saline |
| PL | poly-lysine |
| pNPP | p-Nitrophenyl phosphate |
| RT | Room temperature |
| SDS | Sodium dodecyl sulphate |
| SM | support molecule |
| SMC | smooth muscle cells |
| TX100 | Triton-X100 |

By "culturing" herein, we include culturing single cell types or co-culturing more than one cell type.

As used herein a "positively charged nanoparticle" or "positive nanoparticle" is defined as any particle less than 200 nm, preferably 100 nm or less, that has an overall positive charge. Preferably, the particle is non-toxic, but this is not essential as the particles do not remain with the cells.

As used herein a "negatively charged nanoparticle" or "negative nanoparticle" is defined as any particle less than 200 nm, preferably 100 nm, and most preferably about 2-25 nm, that has an over all negative charge. Preferably, the particle is non-toxic, but this is not essential as the particles do not remain with the cells for a long period of time.

As used herein "magnetically responsive element" can be any element or molecule that will respond to a magnetic field. As detailed below, one of the nanoparticles must contain or be a magnetically responsive element.

As used herein "support molecule" refers to any long molecule that will interact with the nanoparticles to create a mat like fibrous structure or gel and thus hold the magnetic nanoparticle in close proximity with the cell for uptake. One preferred support molecule is polylysine.

By "stem cell" herein, we include toti- and multi-potent cells, unless specifically indicated otherwise.

By "antibody display," we mean that the cell displays the antigen binding site of a particular antigen on its cell surface. However, the entire antibody gene need not be used, and typically is not. Instead it is fused with some other protein, typically a transmembrane protein. Nevertheless, the fusion protein will retain the specific binding properties of an antibody.

By "display cell" what is meant is a cell that displays a surface molecule, in particular one or more ligands, preferably at least one antigen binding site and/or antibody, that can be used to adhere to a target cell.

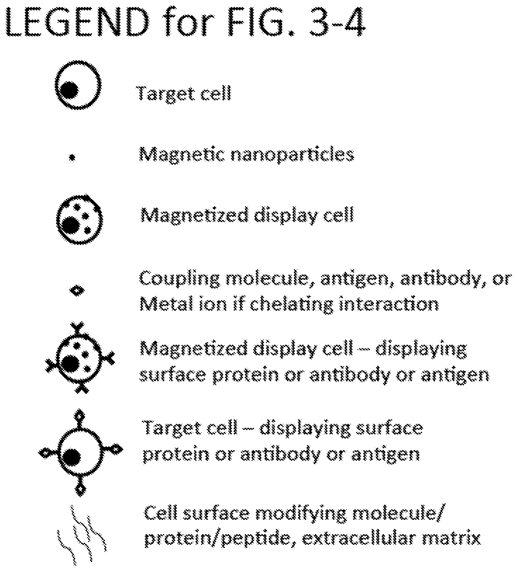

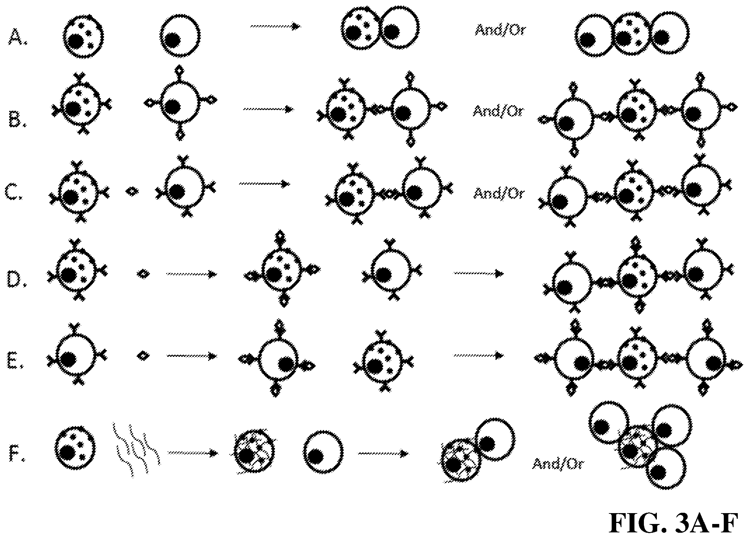

By "display surface marker" or "surface marker" what is meant are those surface displayed markers, chemical or atoms that allow the specific binding to a target cell of interest. Preferred surface molecules include antigen binding sites, antibodies, and the like, but other ligands, receptors, or chemicals could be used as well. The "display surface marker" and its "cognate binding member" are together known as "binding pairs." Other binding pairs are shown in **FIG. 3****,** and listed here:
1. Ligand-receptor interactions
2. Homotypic or heterotypic protein interaction
3. G protein-coupled receptor (GPCR) interactions.
4. EpCam for capturing circulating tumor cells
5. Cell surface receptors
6. Transmembrane proteins
7. Integrins
8. Extracellular matrix proteins, i.e. collagen, laminin, fibronectin
9. Lipid-lipid interaction
10. Lipid-protein interaction
11. Chelating interaction, such as metal ion and histidine polypeptide or other chelating molecules.

By "binding pairs" we mean two atoms or molecules that bind to each other, even where a tertiary member or bridging member is needed.

By "magnetized display cells" what is meant is cells or cell clusters that display a surface molecule that can be used to adhere to a cognate binding member on the surface of a target cell, and which contains therein (or in the ECM or in a surface coating) a sufficient amount of magnetic nanoparticles, such that the cell can be attracted by a magentic field.

In the context of the present invention the term "magnetic cell complex" refers to a complex, in particular an assembly of at least one magnetic display cell and at least one target cell. Thus, a "magnetic cell complex" as used herein refers to an assembly of a single magnetized display cell and a single target cell but also to an assembly of a single magnetized display cells with more than one target cell and vice versa. The term furthermore encompasses an assembly in which at least one magnetic display cell is bound to at least one target cell, which again is bound to at least one other magnetic display cell and so on. The "magnetic cell complex" of the present invention is in particular characterized in that the at least one magnetized display cell displays at least one ligand on its cell surface and the at least one target cell has at least one binding partner on its cell surface, wherein the at least one ligand on the cell surface of the at least one magnetized display cell binds the binding partner on the cell surface of the at least one target cell to form the magnetic cell complex.

As used herein "manipulate" in all of its conjugations refers to any type of interference. In particular, in the context of the present invention the term is used to describe any external act or influence suitable to interfere with cells, in particular target cells. In a specific embodiment "manipulation of target cells" refers to influencing target cells bound to magnetized display cells by application of a magnetic field.

### BRIEF DESCRIPTION OF THE DRAWINGS

**FIG. 1****.** Diagram of an expression plasmid for display of scFv on mammalian cells. PCMVcytomegalovirus promoter; Igκ SP, murine Igκ chain signal peptide; VH, heavy chain variable region; VL, light chain variable region; Linker, a flexible synthetic linker between VH and VL; myc, an epitope tag to measure the scFv expression level; PDGFR, the transmembrane domain of human platelet-derived growth factor receptor; PSV40/ori, SV40 promoter and origin facilitating episomal replication in mammalian cells expressing SV40 large T antigen; Neo/KanR, neomycin- and kanamycin-resistance gene. From Ho, 2009.
**FIG. 2****.** Schematic illustration of surface display on mammalian cells. An additional 10-amino acid epitope tag (c-myc) was fused to the C-terminus of the scFv (based on the anti-CD22 RFB4 Fv structural model), allowing quantitation of fusion display with mAb 9E10 independent of antigen (CD22) binding. Fusion to the N-terminal portion of the PDGFR transmembrane domain was used to anchor scFv on the mammalian (HEK-293T) cell surface. Ho, 2009.
**FIG. 3****.** Schematic illustration of general interactions between magnetized display cells and target cells.
**FIG. 3A****.** Interaction, including protein-protein, lipid-lipid, lipid-protein, electrostatic, Van der Walls. In the case of collecting and assembling cells that exist in low numbers in probed samples, such blood with circulating tumor cells (CTCs) or small tissue biopsy samples. Magnetized display cells can function as supporting or feeding layer to support growth of CTCs or biopsy cells.
**FIG. 3B****.** Interaction between proteins at cell surface, such antibody-antigen or protein-protein interactions.
**FIG. 3C****.** Interaction mediated or triggered by adding a coupling or bridging ligand (molecule or protein or metal ion) which will interact with proteins or antibodies present on the surface of both cell types. These molecules could also be protein, DNA in the form of aptamers and/or metal if a chelating polypeptide, such as polyhistidine, mediates the cell-cell interaction.
**FIG. 3D****.** Similar to C, but here magnetized display cells are incubated with molecules that will be captured by surface protein/antibody, free/unbound molecules are removed, then the magnetized is combined with target cell.
**FIG. 3E****.** Similar to D, but here target cells are incubated with molecule to be captured by its surface protein/antibody, free/unbound molecules are removed, then magnetized display cells are combined with target cell.
**FIG. 3F****.** Magnetized display cells are modified with cell surface modifying molecules or polymers, such as extracellular matrix proteins, poly-L-lysine, fibronectin, collagen, laminin, and the like. The added molecules promote interaction between the magnetized display cell with the target cell.

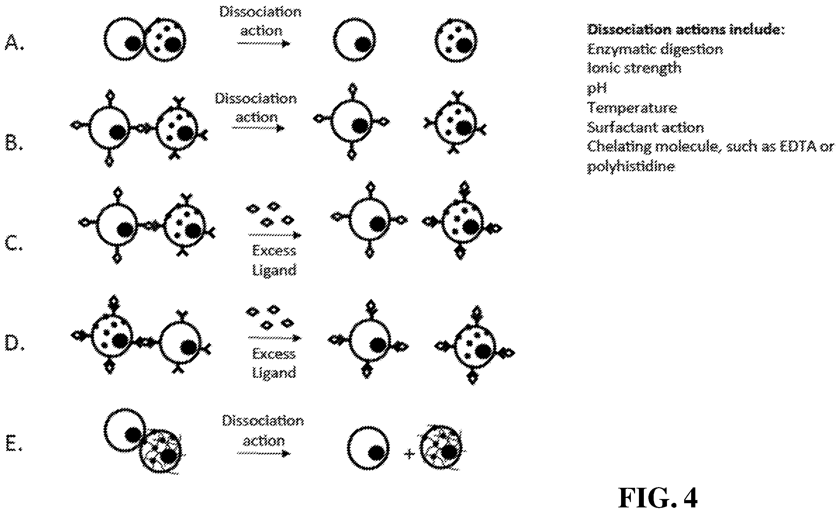

**FIG. 4A-E****.** Schematic illustration of how to dissociate interaction between magnetized display cells and target cells. Dissociation action includes: A. enzymatic digestion, change in ionic strength, change in media composition, change in pH, change in temperature, surfactant action. B., dissociation by one or more dissociation actions. C. Addition of excess ligand. D. Excess ligand or chelating molecule such as EDTA. E. Dissociation by dissociation action.

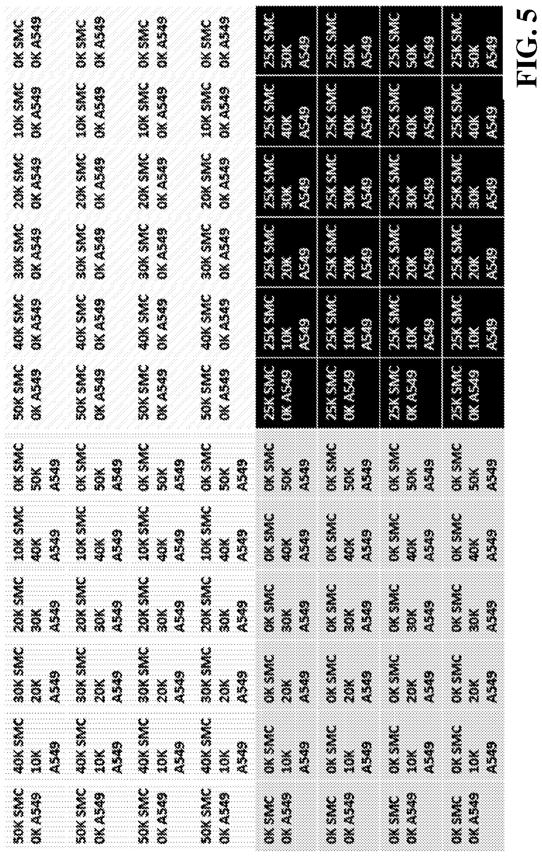

**FIG 5****.** Experimental layout, where primary tracheal smooth muscle cells (SMC) were magnetized (magnetized display cell) and lung cancer adenocarcinoma A549 cells (target cell) were not magnetized. Tracheal SMC are part of pulmonary/lung tissue system, therefore affinity between SMC and lung cancer adenocarcinoma target cell is expected. Number of cells used were: 50K, 40K, 30K, 20K, 10K, and 0 or no cells, as indicated in the plate array labels.

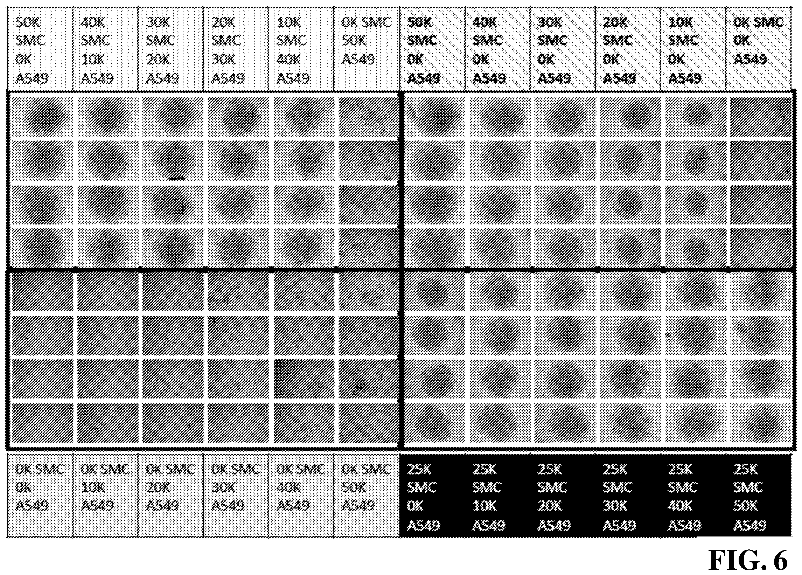

**FIG. 6****.** Photomicrographs (2.5X magnification) of cells according to experimental layout shown in **FIG. 5** at time 0. Time zero is immediately after the two cell types, one magnetized and the other not, are magnetically aggregated or bioprinted at the bottom of a cell repellent 384-well plate after the two cell types were allowed to interact for 15 to 30 minutes.

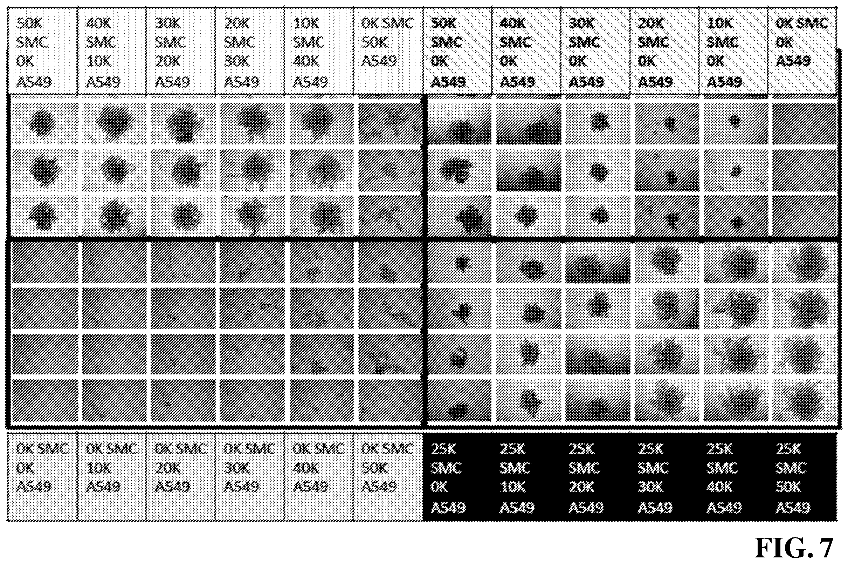

**FIG 7****.** Photomicrographs (2.5X magnification) of cells according to experimental layout listed in **FIG. 5** and shown in **FIG. 6** after 24 hours of culturing these cells.

### DESCRIPTION OF EMBODIMENTS OF THE INVENTION

Generally speaking, the invention is a method of using a magnetic display cell in any of the ways that magnetic beads were previously used. The use of cells, instead of artificial beads, means that no polymers or crosslinking agents or activators need ever be used. Additionally, the use of linkers and associated chemistry is no longer needed, since the magnetized cells already display the relevant antigen binding sites on the cell surface. Further, the cells can be retained, and if fibroblasts, including irradiated fibroblasts, can serve as feeder cells for subsequent cultures.

The targeting sequences preferrably include antigen binding sites, but any cell targeting protein or reagent could be used. As another option, cells that naturally bind to the target cells could be used, e.g., immune cells, cancer cells, smooth muscle cells, edothelial cells, bone cells, epithelial cells, and the like.

In preferred embodiments, the magnetizing materials include positively and negatively charged nanoparticles, one of which must contain one or more magnetically responsive elements, such as nanosized iron oxide. These nanoparticles are further combined with a polymer, preferably a cellular or synthetic polymer, or other long molecule that acts as a support (herein called a "support molecule") for the charged nanoparticles and the cells, holding the nanoparticles in place for their uptake or adsorption by the cells. The inclusion of both positive and negative nanoparticles allows intimate admixing of the nanoparticles and drives the assembly of the three components, thus ensuring even distribution and good uptake. The support molecule intimately combines all three components with the cells in fibrous mat-like structure that allows the cells to take up the magnetically responsive element.

After a period of incubation, the magnetizing material can be washed away, allowing the cells to be manipulated in a magnetic field. The magnetic nanoparticles are eventually lost from the cells by 8 days, but we now know they are retained by the ECM for the duration of the culture.

The magnetically responsive element can be any element or molecule that will respond to a magnetic field, e.g., rare earth magnets (e.g., samarium cobalt (SmCo) and neodymium iron boron (NdFeB)), ceramic magnet materials (e.g., strontium ferrite), the magnetic elements (e.g., iron, cobalt, and nickel and their alloys and oxides). Particularly preferred are paramagnetic materials that react to a magnetic field, but are not magnets themselves, as this allows for easier assembly of the materials.

Preferably, the magnetic field used to levitate such cells or the magnetic ECM is about 300G-1000G. However, the field strength varies with both distance from the culture, and with the amount and type of magnetic response element taken up or adsorbed by the cells. Thus, the optimal field strength will vary, but is easily determined empirically.

The negatively charged nanoparticles include charge stabilized metals (e.g. silver, copper, platinum, palladium), but preferably is a gold nanoparticle.

The positively charged nanoparticles include surfactant or polymer stabilized or coated alloys and/or oxides (e.g. elementary iron, iron-cobalt, nickel oxide), and preferably is an iron oxide nanoparticle.

One of the two nanoparticles must be magnetically responsive, but obviously either one (or both) could contain this feature.

The nanoparticles should have a nano-scale size, and thus are about 50 nm. Size can range, however, between about 5-250 nm, 50-200 nm, 75-150 nm, but they can be smaller or larger or an assembly of nanoparticles, provided only that the size is appropriate to allow entry or adsorption to the cell type in use. Larger particles are less efficient at cell entry. We have shown in other work that there is an upper limit on the effective size of the magnetic nanoparticle, and micrometer size is too big for effectiveness, although some functionality was still observed.

The "support molecule" is generally a polymer or other long molecule that serves to hold the nanoparticles and cells together in an intimate admixture, like a tangled felt mat. The support molecule can be positively charged, negatively charged, of mixed charge, or neutral, and can be combinations of more than one support molecule.

Examples of such support molecules include the natural polymers, such as peptides, polysaccharides, nucleic acids, and the like, but synthetic polymers can also be employed. Particularly preferred support molecules include poly-lysine, fibronectin, collagen, laminin, BSA, hyaluronan, glycosaminoglycan, anionic, non-sulfated glycosaminoglycan, gelatin, nucleic acid, extracellular matrix protein mixtures, matrigel, antibodies, and mixtures and derivatives thereof.

Generally speaking, the concentration of the support molecule is substantially greater than the concentration of the negatively and positively charged nanoparticles, ranging from 10-1000 fold greater, 20-500, or 50-200 fold greater. However, greater or lesser amounts are possible, depending on what cell type is being used and which support molecule and nanoparticles are being used. The longer the polymer, the less may be needed to form sufficient structure to hold the nanoparticles in place for uptake.

Generally, the nanoparticles are used in very low concentrations. Concentrations can range between 10⁻⁶-10⁻¹² Molar, but are preferably in the nanomolar range, and the support molecule(s) 10⁻³-10⁻⁹ Molar, and are preferably in the micromolar range. At least 1 magnetic nanoparticle is needed per cell, but preferably there are hundreds or thousands as more nanoparticles means a lesser field strength in needed.

The three components assemble by electrostatic interaction, and thus charged or mixed charge support molecules, such as poly-lysine, are preferred. However, any of the three components can be functionalized, derivatized, or coated so as to further promote interaction of the components and/or the cells. Thus, one or more members can be functionalized, derivatized, or coated with an antibody that e.g., binds to a cell surface antigen. Thus, interactions between the components and/or the cells would be further promoted. Other binding pairs included receptors-ligands, biotin-strepavidin, complementary nucleic acids, wheat germ agglutinin (WGA), sialic acid containing molecules, and the like.

Coatings can also include protective or passivating coatings, particularly for the nanoparticles, such as PVP, dextran, BSA, PEG, poly-L-lysine and the like. The nanoparticles, especially the nanoparticle that comprises the magnetically responsive element, can be labeled for visualization, e.g., with a fluorophore, radiolabel, or the like, particularly during the development and *in vitro* testing of magnetized cells and tissues. However, for therapeutic uses, it may be preferred to omit such labels.

If desired the magnetic nanoparticle assembly can be made free from biological molecules, such as phage or cell products, because support molecules, such as poly-lysine, can easily be made synthetically. Yet all of the components are generally non-toxic, inexpensive or easy to make. Further, the tangled, fibrous, mat- or felt-like structures allows for the incorporation of additional cell support molecules (such as extracellular matrix components) to be included into the nanoparticle magnetic assemblies.

Magnetizing cells with magnetic nanoparticle assemblies consists of only adding assembly to cells in e.g., regular cell culture media. Cells can be magnetized within minutes from magnetic nanoparticle treatment (5 minutes) and either attached or suspended cells can be treated with magnetic nanoparticle assemblies.

The magnetized antibody diplay cells are then incubated with the target cells, such as blood or a tissue homogenate, and the target cell will then bind to the antigen binding sites of the magnetized cells, allowing their collection in a strong magnetic field. These can be washed one or more times if desired. The collected cells can be used as is or can be further cultured before use.

If it is desired to separate the magnetized display cells from the target cells, some methods are shown in FIG. 4. One could use excess of unbound ligand for competitive binding with cell-cell interactions. Another methods uses enzymes that cleave specific sites of one member of the binding pair, and cell, and such sites could even be engineered into the display molecule. We could also use trypsinization, or general enzymatic digestion with enzyme mixture, such as papain, dispase, collagenase, hyaluronidase, and trypsin, then remove magnetic cells with magnet. A brief temperature "shock" can also dissociate some interactions. Finally, EDTA could also be added if the cell-cell interaction is mediated by chelating proteins or molecule, such as histidine polypeptide.

In a preferred embodiment, the magnetized cells are fibroblast cells or cells from a target organ, and a 3D culture is initiated, using the fibroblasts as feeder cells or cells from a target organ. The 3D culture can be initiated with by the application of a magnetic field, which levitates or bioprints the cells, which quickly coalesce into a 3D structure. The 3D culture shape can also be changed by chaging the shape of the magentic field. The final 3D culture can then be use in cell and tissue therapies, and perhaps even in organ transplantation in the future.

FIG. 5 shows the experimental or plate layout, while FIG. 6 shows the results. FIG. 6 demonstrates that cell type associated with the tissue in which a particular type of cancer grow in vivo, when magnetized can be used to collect and culture the target cancer cells.

The top four rows and six columns (top-left quadrant) were a mixture of the two cell types, where the total number of cells was kept constant (at 50K cells), but the ratio between the two cell types are varied as indicated. The top four rows were replicas of the conditions set for each column to show reproducibility. Columns seven to twelve and top four rows (top-right quadrant) are the controls for the top-left quadrant, where no target cell (lung adenocarcinoma) is present. The bottom four rows and seven to twelve right columns (bottom-right quadrant) the number for magnetized display cell (SMC) is kept constant at 25K cells per well and the number of target cell (A549 adenocarcinoma) varied from 0 to 50K cells, as indicated. The bottom four rows and first six left columns (bottom-left quadrant) there were no magnetized cells (SMC) and the number of target cell (A549 adenocarcinoma) varied from 0 to 50K cells.

More specifically, primary tracheal smooth muscle cells (SMC) are magnetized (magnetized display cell) and allowed to interact with target lung cancer adenocarcinoma A549 (target cell) which is not magnetized. Of the displayed culture wells, only the wells where magnetized display cell were presented generated the magnetically bioprinted circular structures (top-left, top-right, and bottom-right quadrants). The wells in bottom left quadrant (bottom four rows and first six left columns), which only target cells were added, cells were dispersed in the well, a circular pattern was not generated because these cells were not magnetized, therefore were not guided into the magnetic circular pattern.

The protocol for mixing magnetized display cells and nonmagnetized cells is as follows:
1. Detach and count both cells types, one to be magnetized (magnetized display cell) and the other non-magnetized (target cell).
2. Bring both cells types to a concentration of 1 million per milliliter.
3. For the cells to be magnetized, add NanoShuttle at a concentration of 1 µL per 10 thousand cells and centrifuge the mixture 3x at 100G for 5 minutes. Cells are gently resuspended after each centrifugation method. Alternatively, cells can be magnetized overnight incubation of NanoShuttle while in a monolayer culture.
4. Add the desired or unknown amount of the nonmagnetized cells to the well.
5. Add the desired amount of magnetized cells to the well.
6. Add media to the well to bring the total volume to 300 µL.
7. Let cells interact for 15 minutes with gentle swirling of microwell plate every 3 minutes - keep cells in incubator at 5% CO₂, 37°C.
8. Place the plate on the magnet drive for 15 minutes to assemble collected cells together, magnetized and target cells.
9. Remove the plate from the magnet drive and incubate at 5% CO₂, 37°C.

**FIG. 7** shows morphological differences between all four quadrants as a result of the different number of cells per well and ratio between magnetized display cells and target cells (non-magnetized). More specifically, top-right quadrant (magnetized display cells only) show distinct morphology, shape, and size relative to the left-top quadrant with the target cell present. More specifically, in the top-left quadrant, the overall sized is kept nearly the same as the number of magnetized display cells decrease from 50K (left wells) to 0K or no cells (right wells), but size remained nearly the same, with the exception of the wells with no magnetized display cells (0K SMC, right wells). This indicates that the magnetized display cell is collecting and facilitating the growth of the target cell.

Next, in the bottom-right quadrant, where the number of magnetized display cells was kept constant at 25K and the number of target cells (non-magnetized) increased, the 3D structures increased in size as the number of target cells increased. Again, this shows target cells are being collected and facilitated to grow by the magnetized display cells.

Alsberg et al., Magnetically-Guided Self-Assembly of Fibrin Matrices with Ordered Nano-Scale Structure for Tissue Engineering, Tissue Engineering, 2006, vol. 12, pp. 3247-3255.

Bowers et al., Mammalian cell display for the discovery and optimization of antibody therapeutics, Methods. 65(1):44-56 (2014).

Haisler et al., Three-dimensional cell culturing by magnetic levitation (2013), pubished online at Nano3Dbio.com.

Ho & Pastan, Mammalian Cell Display for Antibody Engineering, Methods Mol Biol. 525: 337-xiv (2009).

Ino et al., Cell Patterning Using Magnetite Nanoparticles and Magnetic Force, Biotechnology amd Bioengineering, Aug. 1, 2007, vol. 97, pp. 1309-1317.

Ito et al., Construction of 3D Tissue-Like Structure Using Functional Magnetite Nanoparticles, Yakugaku Zasshi, Jan. 2008, vol. 128 No. 1, pp. 21-28.

Jurgons et al., Drug loaded magnetic nanoparticles for cancer therapy, Journal of Physics: Condensed Matter, 2006, vol. 18, pp. S2893-S2902.

Kuroda et al., Cell surface-engineered yeast displaying a histidine oligopeptide (hexa-His) has enhanced adsorption of and tolerance to heavy metal ions, Appl Microbiol Biotechnol. 57(5-6):697-701 (2001).

Lin Ruei-Zeng et al., Magnetic reconstruction of three-dimensional tissues from multicellular spheroids, Tissue Engineering. Part C, Methods Sep. 2008, 14(3): 197-205.

Lin Ruei-Zhen et al., Recent advances in three-dimensional multicellular spheroid culture for biomedical research, Biotechnology Journal, 2008, 3(9-10): 1172-1184.

Lu et al., Comparison of decellularization techniques for preparation of extracellular matrix scaffolds derived from three-dimensional cell culture, J Biomed. Mater. Res. 00A:000-000 (2012) (article preview available online).

Reilly & Engler, Intrinsic extracellular matrix properties regulate stem cell differentiation, J. Biomech. 43(1): 55-62(2010).

Souza et al., Three-dimensional tissue culture based on magnetic cell levitation, Nature Nanotechnology, Apr. 2010, 5(4): 291-296.
US20150104844 Materials for magnetizing cells and magnetic manipulation
US20020086842 Method for transfecting cells using a magnetic field
US20060063252 Cell culture method and cell sheet
US20060275757 Magnetic nanomaterials and methods for detection of biological materials
US20090137018 Magnetic three-dimensional cell culture apparatus and method US7971592, US8701676, US20060264690, US20100132722, A combination comprising a magnet and a magnetic cell.

US8815231 Systems and methods for magnetic guidance and patterning of materials

14/723,461, MAGNETIC STEM CELL THERAPY FOR LESIONS, also 62/005,383 May 30, 2014, 62/033,017 August 4, 2014.

## Claims

1. A method of isolating target cells from a mixture of cells, comprising:
a) combining a mixture of cells comprising target cells and non-target cells with magnetized display cells that display one or more ligands on their surfaces, said ligands specific for a binding partner on a surface of said target cells;
b) allowing said ligand to bind to said binding partner to produce magnetized display cells bound to said target cells;
c) applying a magnetic field to collect said magnetized display cells bound to said target cells; and
d) washing away non-target cells, thereby isolating said target cells.

2. The method of claim 1, wherein said ligands comprising antigen binding sites and said binding partner comprises an antigen that is recognized by said antigen binding sites.

3. The method of claim 1-2, wherein said magnetized display cells are fibroblasts.

4. The method of claim 1-2, wherein said magnetized display cells are from a fibroblast derived cell line.

5. The method of claim 1-4, wherein said target cells and said magnetized display cells are human.

6. The method of claims 1-5, comprising the further step of 3D culturing said magnetized display cells bound to said target cells.

7. The method of claims 1-5, comprising the further step of 3D culturing said magnetized display cells bound to said target cells in a magnetic field such that said magnetized display cells bound to said target cells are levitated.

8. The method of claims 1-7, wherein said magnetized display cells are provided by treating cells displaying one or more ligands with magnetic nanoparticles.

9. The method of claim 8, wherein said magnetic nanoparticles are introduced into said cells displaying one or more ligands by blasting, injection, electroporation, magnetic pressure, hydrogels, or cationic liposomes.

10. The method of claim 8, wherein said magnetic nanoparticles are introduced into said cells displaying one or more ligands with a composition comprising:
a) a negatively charged nanoparticle;
b) a positively charged nanoparticle; and
c) a support molecule,
d) wherein one of said negatively charged nanoparticle or positively charged nanoparticle is a magnetically responsive element or compound, and wherein said support molecule holds said negatively charged nanoparticle and said positively charged nanoparticle in an intimate admixture forming a fibrous mat-like structure.

11. A method of isolating target cells from a mixture of cells, comprising:
a) combining cells displaying one or more antigen binding site(s) on their surfaces with a composition to form magnetized display cells, said composition comprising:
i) a negatively charged nanoparticle;
ii) a positively charged nanoparticle; and
iii) a support molecule;
iv) wherein one of said negatively charged nanoparticle or positively charged nanoparticle is a magnetically responsive element or compound, and wherein said support molecule holds said negatively charged nanoparticle and said positively charged nanoparticle in an intimate admixture forming a fibrous mat-like structure;
b) combining a mixture of cells comprising target cells and non-target cells with said magnetized display cells;
c) allowing said antigen binding site(s) to bind to one or more target cell(s) displaying an antigen that is recognized by said antigen binding site(s);
d) applying a magnetic field to collect magnetized display cells bound to said target cells; and
e) washing away non-target cells, thereby isolating magnetized display cells bound to said target cells.

12. The method of claim 11, comprising the further step of separating the magnetized display cells from the target cells.

13. The method of claim 11, comprising the further step of 3D culturing said isolating magnetized display cells bound to said target cells in a magnetic field sufficient to levitate said isolating magnetized cells bound to said target cells.

14. The method of claims 11-13, a) wherein the support molecule comprises peptides, polysaccharides, nucleic acids, polymers, poly-lysine, fibronectin, collagen, laminin, BSA, hyaluronan, glycosaminoglycan, anionic, non-sulfated glycosaminoglycan, gelatin, nucleic acid, extracellular matrix protein mixtures, antibody, or mixtures or derivatives thereof, b) wherein said negatively charged nanoparticle is a gold nanoparticle, and c) wherein said positively charged nanoparticle is an iron oxide nanoparticle.

15. A method of manipulating target cells, comprising:
a) combining target cells with magnetized display cells that display one or more ligands on their surfaces, said ligands for specifically binding a target molecule on a surface of said target cells;
b) allowing said ligands to bind to said target molecule to produce magnetized display cells bound to said target cells;
c) applying a magnetic field to collect said magnetized display cells bound to said target cells, thereby allowing manipulation of said target cells.

16. A magnetic cell complex comprising:
a) at least one magnetized display cell displaying at least one ligand on a surface of the at least one magnetized display cell; and
b) at least one target cell having at least one binding partner on a surface of the at least one target cell,
wherein said at least one ligand on the surfaces of the at least one magnetized display cell binds said binding partner on the surface of the at least one target cell so as to form the magnetic cell complex.

17. The magnetic cell complex of claim 16, wherein said at least one ligand comprises antigen binding site(s) and said at least one binding partner comprises an antigen that is recognized by said antigen binding site(s).

18. The magnetic cell complex of claims 16-17, wherein the at least one magnetized display cell comprises magnetic nanoparticles.

## Patentansprüche

1. Verfahren zum Isolieren von Zielzellen aus einem Gemisch von Zellen, umfassend:
a) Kombinieren eines Gemischs von Zellen, das Zielzellen und Nicht-Zielzellen umfasst, mit magnetisierten Displayzellen, die einen oder mehrere Liganden an ihren Oberflächen präsentieren, wobei die Liganden spezifisch für einen Bindungspartner an einer Oberfläche der Zielzellen sind;
b) Erlauben, dass der Ligand an den Bindungspartner bindet, um magnetisierte Displayzellen zu produzieren, die an die Zielzellen gebunden sind;
c) Anlegen eines Magnetfelds, um die magnetisierten, an die Zielzellen gebundenen Displayzellen zu sammeln; und
d) Abwaschen von Nicht-Zielzellen und dadurch Isolieren der Zielzellen.

2. Verfahren nach Anspruch 1, wobei die Liganden Antigen-Bindungsstellen umfassen und der Bindungspartner ein Antigen umfasst, das von den Antigen-Bindungsstellen erkannt wird.

3. Verfahren nach Anspruch 1 bis 2, wobei die magnetisierten Displayzellen Fibroblasten sind.

4. Verfahren nach Anspruch 1 bis 2, wobei die magnetisierten Displayzellen zu einer von einem Fibroblasten abgeleiteten Zelllinie gehören.

5. Verfahren nach einem der Ansprüche 1 bis 4, wobei die Zielzellen und die magnetisierten Displayzellen menschlich sind.

6. Verfahren nach einem der Ansprüche 1 bis 5, umfassend den weiteren Schritt der 3D-Kultivierung der magnetisierten Displayzellen, die an die Zielzellen gebunden sind.

7. Verfahren nach einem der Ansprüche 1 bis 5, umfassend den weiteren Schritt der 3D-Kultivierung der magnetisierten Displayzellen, die an die Zielzellen gebunden sind, in einem Magnetfeld, so dass die an die Zielzellen gebundenen magnetisierten Displayzellen levitiert werden.

8. Verfahren nach einem der Ansprüche 1 bis 7, wobei die magnetisierten Displayzellen bereitgestellt werden, indem Zellen, die einen oder mehrere Liganden präsentieren, mit magnetischen Nanopartikeln behandelt werden.

9. Verfahren nach Anspruch 8, wobei die magnetischen Nanopartikel in die Zellen, die einen oder mehrere Liganden präsentieren, durch Beschießen, Injektion, Elektroporation, magnetischen Druck, Hydrogele oder kationische Liposome eingebracht werden

10. Verfahren nach Anspruch 8, wobei die magnetischen Nanopartikel in die Zellen, die einen oder mehrere Liganden präsentieren, mit einer Zusammensetzung eingebracht werden, die umfasst:
a) einen negativ geladenen Nanopartikel;
b) einen positiv geladenen Nanopartikel; und
c) ein Trägermolekül,
d) wobei eines von dem negativ geladenen Nanopartikel oder dem positiv geladenen Nanopartikel ein magnetisch responsives Element oder eine magnetisch responsive Verbindung ist, und wobei das Trägermolekül das negativ geladene Nanopartikel und das positiv geladene Nanopartikel in einer engen Mischung unter Bildung einer faserigen, mattenartigen Struktur hält.

11. Verfahren zum Isolieren von Zielzellen aus einem Gemisch von Zellen, umfassend:
a) Kombinieren von Zellen, die eine oder mehrere Antigen-Bindungsstellen an ihren Oberflächen präsentieren, mit einer Zusammensetzung, um magnetisierte Displayzellen zu bilden, wobei die Zusammensetzung umfasst:
i) einen negativ geladenen Nanopartikel;
ii) einen positiv geladenen Nanopartikel; und
iii) ein Trägermolekül;
iv) wobei eines von dem negativ geladenen Nanopartikel oder dem positiv geladenen Nanopartikel ein magnetisch responsives Element oder eine magnetisch responsive Verbindung ist, und wobei das Trägermolekül das negativ geladene Nanopartikel und das positiv geladene Nanopartikel in einer engen Mischung unter Bildung einer faserigen, mattenartigen Struktur hält;
b) Kombinieren eines Gemischs von Zellen, das Zielzellen und Nicht-Zielzellen umfasst, mit den magnetisierten Displayzellen;
c) Erlauben, dass die Antigen-Bindungsstelle(n) an eine oder mehrere Zielzellen bindet/binden, die ein Antigen präsentieren, das von der/den Antigenbindungsstelle(n) erkannt wird;
d) Anlegen eines Magnetfelds, um die magnetisierten Displayzellen, die an die Zielzellen gebunden sind, zu sammeln; und
e) Abwaschen von Nicht-Zielzellen und dadurch Isolieren der magnetisierten Displayzellen, die an die Zielzellen gebunden sind.

12. Verfahren nach Anspruch 11, umfassend den weiteren Schritt des Trennens der magnetisierten Displayzellen von den Zielzellen.

13. Verfahren nach Anspruch 11, umfassend den weiteren Schritt der 3D-Kultivierung der isolierenden magnetisierten Displayzellen, die an die Zielzellen gebunden sind, in einem Magnetfeld, das ausreichend ist, um die isolierenden magnetisierten Zellen, die an die Zielzellen gebunden sind, zu levitieren.

14. Verfahren nach einem der Ansprüche 11 bis 13,
a) wobei das Trägermolekül Peptide, Polysaccharide, Nukleinsäuren, Polymere, Poly-Lysin, Fibronectin, Kollagen, Laminin, BSA, Hyaluronan, Glycosaminoglycan, anionisches, unsulfatiertes Glycosaminoglycan, Gelatine, Nukleinsäure, Mischungen von extrazellulären Matrixproteinen, Antikörper, oder Mischungen oder Derivative davon umfasst,
b) wobei der negativ geladene Nanopartikel ein Gold-Nanopartikel ist, und
c) wobei der positiv geladene Nanopartikel ein Eisenoxid-Nanopartikel ist.

15. Verfahren zum Manipulieren von Zielzellen, umfassend:
a) Kombinieren von Zielzellen mit magnetisierten Displayzellen, die einen oder mehrere Liganden an ihren Oberflächen präsentieren, wobei die Liganden für die spezifische Bindung an ein Zielmolekül an einer Oberfläche der Zielzellen dienen;
b) Erlauben, dass die Liganden an das Zielmolekül binden, um magnetisierte Displayzellen zu produzieren, die an die Zielzellen gebunden sind;
c) Anlegen eines Magnetfelds, um die magnetisierten, an die Zielzellen gebundenen Displayzellen zu sammeln, und dadurch die Manipulation der Zielzellen zu erlauben.

16. Magnetischer Zellkomplex, umfassend:
a) zumindest eine magnetisierte Displayzelle, die zumindest einen Liganden an einer Oberfläche der zumindest einen magnetisierten Displayzelle präsentiert; und
b) zumindest eine Zielzelle, die zumindest einen Bindungspartner an einer Oberfläche der zumindest einen Zielzelle aufweist,
wobei der zumindest eine Ligand an den Oberflächen der zumindest einen magnetisierten Displayzelle an den Bindungspartner an der Oberfläche der zumindest einen Zielzelle bindet, um so den magnetischen Zellkomplex zu bilden.

17. Magnetischer Zellkomplex nach Anspruch 16, wobei der zumindest eine Ligand eine oder mehrere Antigen-Bindungsstellen umfasst, und der zumindest eine Bindungspartner ein Antigen umfasst, das von der/den Antigen-Bindungsstellen erkannt wird.

18. Magnetischer Zellkomplex nach einem der Ansprüche 16 bis 17, wobei die zumindest eine magnetisierte Displayzelle die magnetischen Nanopartikel umfasst.

## Revendications

1. Procédé d'isolation des cellules cibles d'un mélange de cellules, comprenant les étapes consistant à :
a) combiner un mélange de cellules, comprenant des cellules cibles et cellules non-cibles, avec des cellules présentatrices magnétisées qui présentent un ou plusieurs ligands sur leurs surfaces, les ligands étant spécifiques d'un partenaire de liaison sur une surface des cellules cibles ;
b) permettre au ligand de se lier au partenaire de liaison pour produire des cellules présentatrices magnétisées liées aux cellules cibles ;
c) appliquer un champ magnétique pour collecter les cellules présentatrices magnétisées liées aux cellules cibles ; et
d) enlever par lavage les cellules non cibles, et ainsi isoler les cellules cibles.

2. Procédé selon la revendication 1, dans lequel les ligands comprennent des sites de liaison à l'antigène et le partenaire de liaison comprend un antigène qui est reconnu par les sites de liaison à l'antigène.

3. Procédé selon la revendication 1 à 2, dans lequel les cellules présentatrices magnétisées sont des fibroblastes.

4. Procédé selon la revendication 1 à 2, dans lequel les cellules présentatrices magnétisées proviennent d'une lignée cellulaire dérivée d'un fibroblaste.

5. Procédé selon la revendication 1 à 4, dans lequel les cellules cibles et les cellules présentatrices magnétisées sont humaines.

6. Procédé selon les revendications 1 à 5, comprenant en outre l'étape de la culture en 3D des cellules présentatrices magnétisées liées aux cellules cibles.

7. Procédé selon les revendications 1 à 5, comprenant en outre l'étape de la culture en 3D des cellules présentatrices magnétisées liées aux cellules cibles dans un champ magnétique, de telle manière que les cellules présentatrices magnétisées liées aux cellules cibles sont lévitées.

8. Procédé selon les revendications 1 à 7, dans lequel les cellules présentatrices magnétisées sont fournies en traitant des cellules qui présentent un ou plusieurs ligands avec des nanoparticules magnétiques.

9. Procédé selon la revendication 8, dans lequel les nanoparticules magnétiques sont introduites dans les cellules qui présentent un ou plusieurs ligands par projection, injection, électroporation, pression magnétique, hydrogels ou liposomes cationiques.

10. Procédé selon la revendication 8, dans lequel les nanoparticules magnétiques sont introduites dans les cellules qui présentent un ou plusieurs ligands avec une composition qui comprend :
a) une nanoparticule chargée négativement ;
b) une nanoparticule chargée positivement ; et
c) une molécule de support,
d) dans lequel une de la nanoparticule chargée négativement et de la nanoparticule chargée positivement est un élément ou un composé magnétiquement responsif, et dans lequel la molécule de support retient la nanoparticule chargée négativement et la nanoparticule chargée positivement dans un mélange intime en formant une structure fibreuse en forme de mat.

11. Procédé d'isolation des cellules cibles d'un mélange de cellules, comprenant les étapes consistant à :
a) combiner des cellules qui présentent un ou plusieurs sites de liaison à l'antigène sur leurs surfaces avec une composition pour former des cellules présentatrices magnétisées, la composition comprenant :
i) une nanoparticule chargée négativement ;
ii) une nanoparticule chargée positivement ; et
iii) une molécule de support ;
iv) dans lequel une de la nanoparticule chargée négativement et de la nanoparticule chargée positivement est un élément ou un composé magnétiquement résponsif, et dans lequel la molécule de support retient la nanoparticule chargée négativement et la nanoparticule chargée positivement dans un mélange intime en formant une structure fibreuse en forme de mat ;
b) combiner un mélange des cellules comprenant des cellules cibles et des cellules non-cibles avec les cellules présentatrices magnétisées ;
c) permettre au(x) site(s) de liaison à l'antigène de se lier à une ou plusieurs cellules cibles présentant un antigène qui peut être reconnu par le ou les sites de liaison à l'antigène ;
d) appliquer un champ magnétique pour collecter des cellules présentatrices magnétisées liées aux cellules cibles ; et
e) enlever par lavage les cellules non-cibles, et ainsi isoler les cellules présentatrices magnétisées liées aux cellules cibles.

12. Procédé selon la revendication 11, comprenant en outre l'étape de séparation des cellules présentatrices magnétisées des cellules cibles.

13. Procédé selon la revendication 11, comprenant en outre l'étape de la culture en 3D des cellules présentatrices magnétisées isolant qui sont liées aux cellules cibles dans un champ magnétique suffisant pour leviter les cellules présentatrices magnétisées isolant qui sont liées aux cellules cibles.

14. Procédé selon les revendications 11 à 13,
a) dans lequel la molécule de support comprend des peptides, des polysaccharides, des acides nucléiques, des polymères, de la poly-lysine, de la fibronectine, du collagène, de la laminine, du BSA, de l'hyaluronane, du glycosaminoglycane, du glycosaminoglycane anionique non sulfaté, de la gélatine, de l'acide nucléique, des mélanges de protéines de la matrice extracellulaire, un anticorps ou des mélanges ou dérivés de ceux-ci,
b) dans lequel la nanoparticule chargée négativement est une nanoparticule d'or, et
c) dans lequel la nanoparticule chargée positivement est une nanoparticule d'oxyde de fer.

15. Procédé de manipuler des cellules cibles, comprenant les étapes consistant à :
a) combiner des cellules cibles avec des cellules présentatrices magnétisées qui présentent un ou plusieurs ligands sur leurs surfaces, les ligands étant adaptés pour la liaison spécifique d'une molécule cible sur une surface des cellules cibles ;
b) permettre aux ligands de se lier à la molécule cible pour produire des cellules présentatrices magnétisées liées aux cellules cibles ;
c) appliquer un champ magnétique pour collecter les cellules présentatrices magnétisées liées aux cellules cibles, ainsi permettant la manipulation des cellules cibles.

16. Complexe cellulaire magnétique, comprenant :
a) au moins une cellule présentatrice magnétisée qui présente au moins un ligand sur une surface de la au moins une cellule présentatrice magnétisée ; et
b) au moins une cellule cible ayant au moins un partenaire de liaison sur une surface de la au moins une cellule cible,
dans lequel le au moins un ligand sur les surfaces de la au moins une cellule présentatrice magnétisée lie le partenaire de liaison sur la surface de la au moins une cellule cible pour former le complexe cellulaire magnétique.

17. Complexe cellulaire magnétique selon la revendication 16, dans lequel le au moins un ligand comprend un ou plusieurs sites de liaison à l'antigène et le au moins un partenaire de liaison comprend un antigène qui peut être reconnu par le ou les sites de liaison à l'antigène.

18. Complexe cellulaire magnétique selon les revendications 16 à 17, dans lequel la au moins une cellule présentatrice magnétisée comprend des nanoparticules magnétiques.
